# EUROPEAN PATENT APPLICATION

(11) **EP 1 726 661 A1**
(43) Date of publication of application: **29.11.2006**
(21) Application number: 05024584.4
(22) Date of filing: 10.11.2005
(51) Int. Cl.: C12Q 1/68, C12N 15/00

(54) **Method for manufacturing a biosensor element**

(30) Priority: 27.05.2005 JP 2005186164
(71) Applicant: HITACHI SOFTWARE ENGINEERING CO., LTD., Yokohama-shi, Kanagawa 230-0045 (JP)
(72) Inventor: Nakahara, Miwako Hitachi, Ltd Intel. Prop. Office, 1-chome Chiyoda-ku Tokyo 100-8220 (JP); Inoue, Takashi Hitachi, Ltd Intel. Prop. Office, 1-chome Chiyoda-ku Tokyo 100-8220 (JP); Saeki, Tomonori Hitachi, Ltd Intel. Prop. Office, 1-chome Chiyoda-ku Tokyo 100-8220 (JP); Kogi, Osamu Hitachi, Ltd Intel. Prop. Office, 1-chome Chiyoda-ku Tokyo 100-8220 (JP); Ban, Noriko Hitachi, Ltd Intel. Prop. Office, 1-chome Chiyoda-ku Tokyo 100-8220 (JP)
(74) Representative: Strehl Schübel-Hopf & Partner

(57) **Abstract**

When probe biomolecules are immobilised on a substrate surface, a surfactant (phase transfer catalyst) is added for reaction, whereby the immobilisation efficiency of the probe biomolecules and the coating uniformity thereof are improved. Consequently, it is possible to dramatically improve the quantitativity and reproducibility of the biosensor element.

## Description

### Background of the Invention

The present invention relates to a method for manufacturing a biosensor element on a surface where nucleic acids, proteins, and the like are immobilised for sensing biomolecules and chemical reactions.

The human genome sequence has been entirely deciphered by the human genome project, and currently a subject matter of the study is shifting from the conventional "sequence analysis" to "functional analysis" that examines functions thereof. Data obtained from this functional analysis are considered to be able to provide a significant clue to elucidate a life phenomenon and it is expected that the functional analysis may become a key to solve a problem in every field associated with living things, such as medical practice, environment, and food.

In the functional analysis above, it is demanded that a gene having enormous amounts of information and a protein made from the gene are analysed exhaustively and rapidly. Considering the above situation, a biochip has been developed, which is a kind of biosensor element, typified by DNA microarrays and protein chips.

There are mainly two methods to manufacture the biochip. One is a method in which an amino acid or a nucleic acid base is chemically bonded sequentially one by one on a substrate by means of photolithography or ink-jet, whereby probe biomolecules such as proteins or on-strand DNAs (deoxyribonucleic acid) are synthesised on the substrate in-situ (see US 5,424,186). The other is a method in which the probe biomolecules are synthesised ex-situ and subsequently immobilised on the substrate (US 5,700,637).

It is expected that the biochip will be used in the future, for example, in medical diagnosis such as diagnosing cancer. If the biochip is used in the medical diagnosis, it is necessary that data obtained from the biochip have a high reliability. In the method where the probe biomolecules are synthesised on the substrate in-situ by means of photolithography or ink-jet, if there is an error in the type of the nucleic acid base and/or the amino acid to be synthesised, or any defect occurs therein, it is impossible under existing circumstances to examine such error or defect, and remove those parts after manufacture. Furthermore, an enormous production cost is required to obtain a long DNA and/or protein.

On the other hand, in the method where DNAs and proteins are synthesised ex-situ and then immobilised on the substrate, a refining process of the synthesised biomolecules can be performed after synthesis. Therefore, it is possible to remove biomolecules having a defect or the like in advance. Accordingly, probe biomolecules of a high degree of purity can be immobilised on the surface, thereby enabling the manufacture of a highly reliable chip. In many instances in this method, biomolecules having reactive groups react with the surface, and the immobilisation is performed by forming a covalent binding. At this stage, photochemical reaction may be used.

However, a chip obtained by immobilising on the substrate surface, a polymer of higher molecular weight, such as DNAs and proteins, may be easily subjected to an uncontrollable number of variations in amount and/or structure of the probe biomolecules being immobilised, with the result that these variations may reduce the reproducibility of data (Nature Vol. 21, pp. 5 - 9, 1999). In addition, when biomolecules are detected by use of the chip, the biomolecules may be non-specifically adsorbed in the substrate surface; the non-specifically adsorbed biomolecules may lower the S/N ratio of the detection. Furthermore, the low density of the probe biomolecules being immobilised may cause low sensitivity. These effects render a quantitative analysis difficult (Nature Biotech. 19, p.342, 2001).

The following three points are major causes of the above problems:
1) Reaction efficiency in immobilisation is low, when the probe biomolecules synthesised ex-situ are immobilised on the chip surface;
2) Reaction active site on the chip surface is not uniform; and
3) Non-specifically adsorbed biomolecules remains on the surface.

One of the causes of 1) above is that while the substrate surface is hydrophobic in many cases, the probe biomolecule is comparatively hydrophilic, and thus affinity between the surface and the biomolecules is low. One of the causes of 2) is that the coating film for producing the reaction active sites on the substrate surface is not uniform. One of the causes of 3) is that non-specifically adsorbed biomolecules adhere to the surface and are hard to remove therefrom.

### Summary of the Invention

The problems described above can be solved by providing a novel coating method which allows probe biomolecules to be immobilised efficiently and uniformly and further suppresses a non-specific adsorption of the probe biomolecules. In particular, it is considered that the problems can be solved by providing a coating method, in which
1) the affinity between the probe biomolecules and the substrate surface is increased,
2) the reaction active sites of the surface are made uniform, and
3) the non-specifically adsorbed biomolecules are hard to adhere to the surface and are easily removed therefrom.

Rickman et al., have been conducting a study of coating reaction solutions used for uniformly immobilising probe cDNAs, when the probe cDNAs are immobilised by a photochemical reaction with UV light. As a result of the study, Rickman et al., have reported that when a solution obtained by adding 3-[(3-cholamidopropyl)-dimethylammonio]-1-propane sulfonate in formamide or dimethyl sulfoxide (DMSO) solution is used as a solution for allowing the probe cDNAs to react, the immobilised amount is increased and also uniformity and reproducibility are improved (Nucleic Acids Research Vol. 31, No. 18 e109 (2003)).

Rickman et al. employed UV light for immobilisation, but when UV light is used, there is a concern that the probe DNAs may be damaged largely, thereby producing degenerative changes in the probe DNAs. Therefore, it is desirable that immobilisation is performed without using UV light. The present invention provides a method which immobilises probe biomolecules efficiently and uniformly without deteriorating the quality of the biomolecules.

A first aspect of the present invention provides a method for manufacturing a biosensor element where probe biomolecules for detecting a biochemical reaction are immobilised on a substrate, wherein a surfactant is added for reaction when the probe biomolecules are immobilised on the substrate.

As a second aspect of the present invention, if the probe biomolecules are nucleic acid, the surfactant is a positively charged surfactant.

As a third aspect of the present invention, if the probe biomolecules are protein and the effective charge of the protein molecules is negative, a positively charged surfactant is used as the surfactant, whereas a negatively charged surfactant is used if the effective charge of the protein molecules is positive.

As a fourth aspect of the present invention, the concentration C of the surfactant is 0.1 CMC = C = 100 CMC (CMC: critical micelle concentration).

As a fifth aspect of the present invention, the concentration C of the surfactant is at least 1 CMC.

As a sixth aspect of the present invention, when the probe biomolecules are immobilised in the biosensor element and the biomolecules are immobilised through the intermediary of reactive groups-containing silane coupling agent molecules, the water content of the reaction solution when the silane coupling agents are allowed to react with the substrate surface is at least 10%, and the reaction time is equal to or less than one hour.

As thus described, in the present invention, it is possible efficiently and uniformly to immobilise biomolecules serving as a probe of the biosensor element on the substrate, and it is further possible to reduce the amount of non-specifically adsorbed biomolecules.

Therefore, the sensitivity of the biosensor element can be largely improved, and further, it is also possible to enhance the quantitativity and reproducibility of the biosensor element.

Accordingly, it is possible to conduct a highly accurate/reliable gene/protein inspection and the like, by means of a small amount of inspection sample.

### Brief Description of the Drawings

These and other features, objects and advantages of the present invention will become more apparent from the following description when taken in conjunction with the accompanying drawings wherein:
- FIG. 1: is a procedural flow chart to explain a process for manufacturing a biosensor element.
- FIG. 2: includes conceptual diagrams indicated by numerals (1), (2), and (3) for explaining the state of the substrate surface, wherein (1) shows an aminated state (first layer), (2) shows a state where reactive groups are introduced (second layer), and (3) shows a state where probe biomolecules are immobilised.
- FIG. 3: includes state diagrams indicated by numerals (1), (2), and (3) for specifically explaining the conceptual diagrams shown in FIG. 2, wherein (1) shows an aminated state (first layer), (2) shows a state where reactive groups are introduced (second layer), and (3) shows a state where probe biomolecules are immobilised.
- FIGs. 4A and FIG. 4B: are schematic diagrams which explain the contribution of surfactant to the reaction when probe DNAs are immobilised.
- FIG. 5: is a chart showing the relationship between the CTAB (Cetyltrimethylammonium bromide) concentration in the reaction solution when the probe DNAs are immobilised, and the DNA amount being immobilised.
- FIG. 6: is a chart which shows the CTAB concentration in the reaction solution when the probe DNAs are immobilised, and the in-array uniformity of the immobilised probe DNAs.
- FIG. 7: is a chart showing the relationship between the CTAB concentration in the reaction solution when the probe DNAs are immobilised, and the ratio of DNA which has been specifically adsorbed.
- FIGs. 8A: and 8B are illustrations showing spotting shapes when the probe DNAs are immobilised.
- FIG. 9: is a chart showing the relationship between hybridisation amount and the CTAB concentration in the reaction solution when the probe DNAs are immobilised.
- FIG. 10: is a schematic block diagram showing the structure of the probe DNAs immobilised on the substrate.
- FIG. 11: is a schematic diagram showing a beads array.

### Detailed Description of Preferred Embodiments

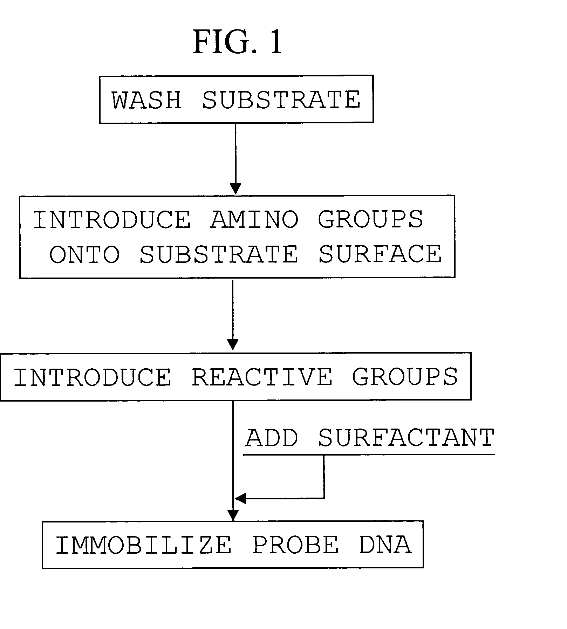

FIG. 1 is a process chart showing a method for manufacturing a biosensor element to which one embodiment of the present invention has been applied. The method for manufacturing the biosensor element according to the present embodiment, includes;
(1) a process for washing a carrier (substrate, beads, or the like),
(2) a process for introducing amino groups onto the surface of the carrier,
(3) a process for introducing reactive groups, and
(4) a process for immobilising probe DNAs by use of surfactant.

Hereinafter, each of the above processes will be explained as the following.

### (1) Process for washing the carrier

A carrier according to the given purpose is prepared and washed. Specifically, for instance, the carrier is washed with an alkaline aqueous solution such as NaOH aqueous solution, and then washed with acid aqueous solution such as HC1 aqueous solution, rinsed with purified water and subsequently dried under reduced-pressure.

For example, a glass substrate (slide glass), quartz substrate, plastic substrate, or the like can be used as the carrier. In addition, a metal coating substrate or the like may be available for the carrier. It is preferable that the material of the carrier is one which has silanol groups on the surface.

It is not necessary that the carrier is of a plane type. For example, the carrier may be in the form of beads, fibres, powder, or the like. When the carrier is in the form of beads, plastic beads such as polystyrene, metal coating beads, magnetic beads, or the like may be employed. As the cleaning solution, a mixed solution of sulphuric acid and hydrogen peroxide may be used.

### (2) Process for introducing amino groups

Silane coupling agents having amino groups are allowed to react with the carrier surface which has been washed, and the amino groups are immobilised on the carrier surface.

As the silane coupling agents, for instance, 3-aminopropyltrimethoxysilane, 3-aminopropyltriethoxysilane, N-(2-aminoethyl) -3-aminopropyltrimethoxysilane), (aminoethylaminomethyl) phenethyltrimethoxysilane, or the like may be used.

Ethanol, methanol, toluene, water, or the like can be used as the solvent, for instance. The reaction temperature is generally, in the range of 25 - 85 °C.

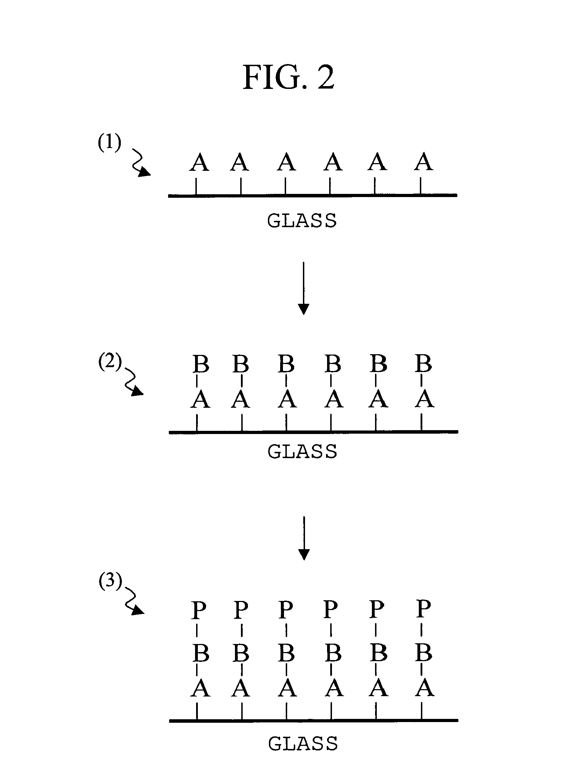

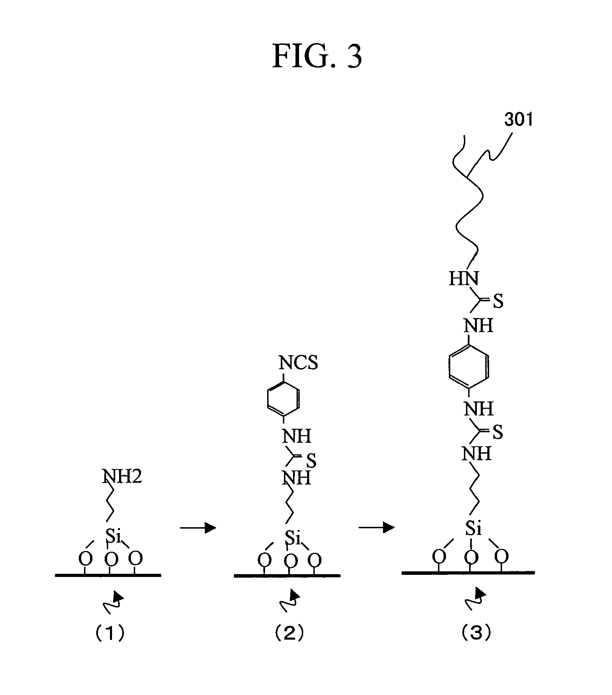

FIG. 2, numeral (1) shows a state of the carrier surface (which is a glass in FIG. 2), where code "A" indicates a molecule immobilised on the first layer according to the above process. There exists an amino group on the terminal of A. In FIG. 3, numeral (1) shows 3-aminopropyl triethoxysilane that is used as the silane coupling agent.

### (3) Process for introducing reactive groups

A compound having reactive groups is allowed to react with the amino groups on the carrier surface, and the reactive groups are immobilised on the carrier surface.

As the compound having reactive groups, PDC (Phenylenediisothiocyanate) having isothiocyanate groups on the terminal, or the like, DSG (Disuccinimidyl glutarate) having soccinimde groups, or the like, KMUS (N-(11-maleimidoundecanoyloxy) succinimide) having maleimide groups, or the like can be employed. Here, the terminal isothiocyanate groups, soccinimde groups, or maleimide groups are referred to as "reactive groups".

DMF (N,N-Dimethyolformamide), DMSO (Dimethylsulfoxide), ethanol, pyridine, or the like can be used as the solvent, for instance. The reaction temperature is generally in the range of 25 - 85 °C.

FIG. 2, numeral (2) shows a state of the carrier surface, where code "B" indicates a part of the reactive group immobilised on the second layer according to the above process. In FIG. 3, numeral (2) shows the PDC that is used as a compound having the reactive groups.

### (4) Immobilisation of probe DNA

The reactive groups formed in the carrier surface are allowed to react with probe DNAs; the terminal of the probe DNAs has a group that is capable of reacting with the reactive groups formed on the carrier surface, whereby the probe DNAs are immobilised on the carrier surface. At this timing, in order to enhance the reactive efficiency of the immobilisation of the probe DNAs, a surfactant is added to the reaction solution.

The surfactant to be added, which works as a phase transfer catalyst, will be explained. In a two-phase system, water phase and oil phase, the surfactant accelerates the transfer of ions between the two phases, the ions dissolved in the water phase and the ions dissolved in the oil phase. In other words, the frequency of collisions between molecules dissolved in respective phases is increased, thereby enhancing the efficiency of the reaction between the two phases.

Though there are two types of surfactant, non-ionic and ionic, it is preferable to use an ionic surfactant. It is further preferable to use a positively charged surfactant.

Such surfactant as mentioned above may include, CTAB(Cetyltrimethylammonium bromide) which is also a transfer catalyst; CₙH₂ₙ₊₁NH₃(Cl⁻) or CₙH₂ₙ₊₁NH₃(Br⁻) (n is at least 8), which is an ammonium salt; CₙH₂ₙ₊₁N(CH₃)₃(Cl⁻) or CₙH₂ₙ₊₁N(CH₃)₃(Br⁻) (n is at least 8); CₙH₂ₙ₊₁N(C₃H₇)₃(Br⁻) (n is at least 14); C₁₈H₃₇N(CH₃)₃(NO₃⁻), C₁₈H₃₇N(CH₃)₃(FO₃⁻), C₁₈H₃₇N(C₂H₅)₃(BrO₃⁻), C₁₈H₃₇N(C₃H₇)₃(BrO₃⁻). C₁₈H₃₇N(C₄H₉)₃(BrO₃⁻); or other than above, tetraalkylammonium salt, trialkylphenylammonium salt, trialkylbenzylammonium salt, alkylpyridinium halides, and the like are taken as examples. Any of the surfactants listed above may be used alone or in a mixture of at least two types thereof.

It is preferable that the added amount of surfactant provides a concentration around the critical micelle concentration (referred to as "CMC"). Here, the "critical micelle concentration" will be explained. The critical micelle concentration is a concentration where the correlation between the concentration of the surfactant and the surface tension of the solution becomes discontinuous, when the surfactant is added to the solution. When the concentration is equal to or less than the critical micelle concentration, the surfactant molecules exist in the form of a monomer in the solution or on the surface of the carrier (substrate). On the other hand, within the region of the critical micelle concentration, the surfactant molecules become an aggregate to form a lump (micelle). Further on the carrier (substrate), the surfactant molecules form a monolayer. As thus described, the behaviour of the surfactant molecules changes drastically around the critical micelle concentration.

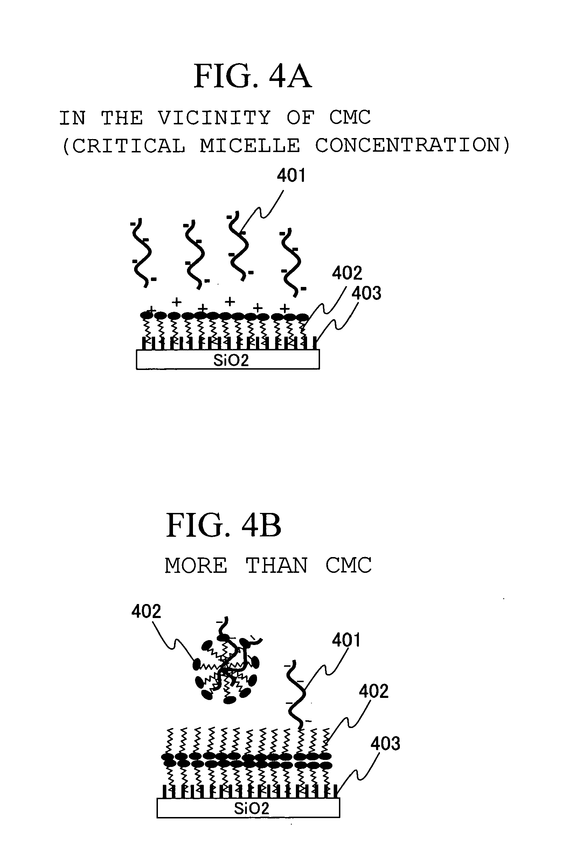

FIG. 4A shows a mechanism how the amount of the immobilised probe DNAs is increased, in the case where a positively charged surfactant is added at a concentration in the vicinity of the critical micelle concentration. In the vicinity of the critical micelle concentration, the positively charged surfactant 402 is adsorbed as a single layer on the reactive groups 403, and negatively charged probe DNA 401 is captured by Coulomb forces. The surfactant 402 is a phase transfer catalyst which improves the affinity between the hydrophobic part and the hydrophilic part. In other words, the concentration of the probe DNAs adjacent to the carrier surface can be increased, and further the collision frequency between the probe DNA (hydrophilic part) and the reactive group on the substrate (hydrophobic part) can be increased.

On the other hand, as shown in FIG. 4B, when the concentration is more than the critical micelle concentration, a multilayered adsorption of the surfactant and/or the creation of micelle may occur on the substrate surface. The adsorbed multilayer may block a diffusion of the probe DNA onto the surface, and the micelle may trap the probe DNAs in the fluid. Therefore, the multilayered adsorption of the surfactant and/or the micelle may work toward blocking the reaction between the probe DNAs and the reactive groups on the substrate.

However, the multilayer and the micelle are not rigidly structured, but are structured dynamically where association/dissociation coexist. Therefore, the characteristics of the phase transfer catalysts are maintained. Therefore, although the reaction efficiency is lower as compared with the condition where the concentration is around the critical micelle concentration, it is enhanced if compared with the condition where the surfactant is not added.

Furthermore, there is a feature that in the concentration region equal to or more than the critical micelle concentration, the reacting amount of the probe DNAs on the substrate surface is stable even if the concentration of the surfactant is changed. This feature is advantageous from the viewpoint of building a robust process.

As a specific concentration of the surfactant, it is desirable to add the surfactant in a concentration of at least 0.1 CMC (in the case of CTAB, 0.08 mM). If the concentration is equal to or more than 0.1 CMC, it is possible to reduce the ratio of non-specifically adsorbed probe DNAs. On the other hand, if the concentration is set high, such as more than 100 CMC (for the case of CTAB, 80 mM), the reaction efficiency falls down as described above. Furthermore, the wettability between the substrate surface and the reaction solution is extremely increased, and thus the spot shape may easily become distorted instead assuming the desired symmetric circular form. Therefore, it is desirable for the concentration to be equal to or less than 100 CMC.

It is to be noted that the amount of solution which is spotted for immobilising the probe DNAs may be around a few pL per spot. Even under highly humid circumstances, there is a case that moisture in the solution evaporates and the concentration of the surfactant may vary depending on the spot. In this case, it is desirable that the immobilised amount of probe DNAs is not varied drastically according to the change of the surfactant concentration.

The concentration region of the surfactant, which may not drastically change the immobilised amount of probe DNAs, is at least 1 CMC (for the case of CTAB, 0.8 mM), and preferably, at least 10 CMC (for the case of CTAB, 8 mM). In other words, it is desirable to add the surfactant within this concentration range, in order to immobilise the probe DNAs in a range with little change according to the concentration change of the surfactant, to improve the reaction efficiency and to reduce the amount of probe DNAs which are adsorbed non-specifically.

The solution in which the probe DNAs are to be dissolved may include a weakly alkaline aqueous solution, such as a carbonic acid buffer and a phosphoric acid buffer. Then, the probe DNAs are dissolved into this solution, and further the surfactant is added to obtain the reaction liquid.

If the carrier is a glass substrate, the reaction liquid in which the probe DNAs are dissolved may be spotted on the surface of the substrate.

If the carrier is in the form of beads, the beads may be soaked in the reaction liquid in which the probe DNAs are dissolved.

The reaction temperature is generally in the range from 25 to 40 °C. The reaction time is in the range from 2 to 12 hours. The reaction is made to occur under the circumstances where humidity is maintained sufficiently, so that the solution may not be dried during the reaction.

With the processing described above, when the reactive groups are isothiocyanate groups or succinimide groups, probe DNAs having an amino group on the 5'-terminal can be immobilised. Alternatively, if the reactive groups are maleimide groups, probe DNAs having a thiol group on the 5'-terminal can be immobilised.

In FIG. 2, numeral (3) shows a state of the carrier surface where "P" represents the probe DNA described above. In FIG. 3, numeral (3) shows a case where the reactive groups are isothiocyanate groups and the probe DNA having the amino group on the 5'-terminal is immobilised.

Up to this point, one embodiment of the present invention has been applied to a method for manufacturing a biosensor element.

In the above embodiment, explanation has been made focusing on the process of immobilising the probe DNAs. In the manufacturing steps above, however, another process may be performed to improve the efficiency and uniformity of immobilisation of lower layers of the probe DNA (layers corresponding to A and B in FIG. 2), in order that the probe DNAs can be immobilised efficiently and uniformly.

By way of example, in the process for introducing the amino groups, when the substrate surface is coated with the first layer (part A of FIG. 2), the water content of the reaction solution and the reaction time are adjusted appropriately.

Specifically, it is preferable to set the water content of the reaction solution containing silane coupling agents, at 10% or more. If the water content of the reaction solution is small, such as less than 10%, this may deteriorate the uniformity and reduce the volume of immobilised probe DNAs. In the range where the water content is large, such as 10% or more, the uniformity in immobilisation of probe DNAs is improved, and the immobilised amount of DNA is increased.

The aforementioned results will be obtained due to the following reasons. When the silane coupling agents react with the surface, silanol groups of the agents react with the substrate surface; those silanol groups are generated by hydrolysis of methoxy groups, ethoxy groups, or the like of the silane coupling agents. If the substrate is made of glass, for instance, siloxane bonds (Si-O-Si) are formed. If the water content is small in amount, the silanol groups are hardly generated. Therefore, amino groups in the silane coupling agents, positively charged, may be dominantly adsorbed in the substrate surface due to the negative charge of the surface, and this may interfere with the reaction.

On the other hand, when the water content is large, the methoxy group part or the ethoxy group part in the silane coupling agents may be swiftly changed to silanol groups via the hydrolysis reaction, and a reaction proceeds rapidly, in which a siloxane bond is formed by reacting the silanol groups and the surface of the substrate. In addition, silanol polymerisation reaction generates water, and thus if the water content in the reaction solution is large, the silanol polymerisation reaction is suppressed. Accordingly, if the water content is large, amination can be performed uniformly, because of the synergistic effect described above.

In the meantime, it is preferable that the reaction time between the carrier and the silane coupling agents is less than one hour. If the reaction time is one hour or more, the uniformity may be deteriorated and the immobilised amount may be reduced. A cause for the above result is considered to be that if the reaction time is long, the polymerisation reaction among the silane coupling agents may proceed, and an aggregate as a product of the polymerisation reaction may adhere on the substrate surface.

Consequently, in the process for introducing the amino groups, it is desirable to perform the process under the circumstances that the reaction time is less than one hour, and the water content of the reaction solution is 10 % or more. From the viewpoint of actual operability, it is preferable to set the reaction time from one minute to one hour, and the water content of the reaction fluid is set to 10% to 50%.

In the above embodiment, DNA has been used as biomolecule, but another biomolecule such as RNA (ribonucleic acid), protein, PNA, sugar chain, or a composite of those elements may also be available.

When a protein is employed and the protein is negatively charged in an aqueous solution, it is desirable to use a positively charged surfactant as a surfactant to be added to the immobilisation solution. On the other hand, if the protein is positively charged in the aqueous solution, it is desirable to use a negatively charged surfactant. The negatively charged surfactant may be, for instance, sulfate esters, sulfonate, alkyl benzene sulfonate, carboxylate, and the like. If the negatively charged surfactant described above is used, the probe immobilisation efficiency and uniformity may be improved.

Furthermore, in the above embodiment, the amino groups are firstly introduced on the carrier surface by the use of silane coupling agents. However, similar effects can be obtained by adding the surfactant described above, also in the case where the molecules having carboxyl group, epoxy group, and the like are immobilised, or in the case where the active groups are immobilised with a coating of avidin.

By use of the immobilisation method described above, it is possible to form a chip surface where the density of the probe biomolecules is 2 × 10¹² molecules/cm² or more, and the ratio of the probe biomolecules which are non-specifically adsorbed and which are not bonded by forming a covalent binding, is 10% or less.

### EXAMPLES

Next, examples of the present invention will be explained in detail. It is to be noted, however, that the present invention is not limited to the following examples.

In the following, examples of the manufacturing method according to the present invention, which utilise plane-type DNA microarray and beads array, will be described.

### <EXAMPLE 1> Immobilisation of linker molecule (molecule having reactive groups) onto a substrate

A slide glass made of borosilicate glass was prepared as a carrier. According to the process shown in FIG. 1, the substrate was washed in NaOH aqueous solution, further washed in HCl aqueous solution, and then, rinsed with purified water. Subsequently, it was dried under reduced pressure. 3-aminopropyltrimethoxysilane which serves as the silane coupling agent was allowed to react with the substrate surface having been washed, and the substrate surface was aminated (see FIG. 3, numeral (1)). In addition, methanol was used as the solvent, and the concentration of the silane coupling agents was 3% (Volume/Volume). The reaction temperature was room temperature, and the reaction time was five minutes.

Next, the aminated substrate was acted upon by PDC (Phenylene diisothiocyanate) having isothiocyanate groups on the terminal. Here, DMF(*N,N*-dimethylformamide) was used as the solvent, and the concentration of PDC was 0.6% (Weight/Volume). The reaction temperature was room temperature (around 15°C to 30°C), and the reaction time for that temperature was 12 hours. It is to be noted that any reaction temperature is applicable as far as it induces a reaction, and the range from 4°C to 40°C is sufficient. Accordingly, a substrate was obtained, on which the reactive groups (linker molecules) were immobilised (see FIG. 3, numeral (2)).

### <EXAMPLE 2> Preparation of reaction liquid containing probe DNA

100 iM of 50-mer probe DNA having a sequence of 50 bases was dissolved in a weak alkaline carbonic acid buffer (1 M Na₂CO₃ and 1M NaHCO₃ are mixed and adjusted to pH 9.0). This solution was preparatively isolated, and CTAB (Cetyltrimethylammonium bromide) serving as positively charged surfactants, with various concentrations from 0 mM to 80 mM, were respectively added to the thus isolated solutions.

It has been reported that the critical micelle concentration (CMC) of CTAB is 0.8 mM (Langumuir Vol. 1 No. 3, p.352, 1985). Therefore, according to the above process, a solution has been obtained which was added with CTAB having a concentration range close to the critical micelle concentration.

### <EXAMPLE 3> Immobilisation of probe DNA onto the substrate

On a plurality of substrates obtained in Example 1, the reaction solutions prepared in Example 2, being different in concentration, were respectively spotted, and substrates with the probe DNA being immobilised were obtained. Here, the reaction temperature was 25 °C, and the reaction time was four hours. The reaction was allowed to occur under the circumstances where humidity was maintained sufficiently, so that the solutions would not dry during the reaction.

### <Example 4> Evaluation

In order to measure the immobilised amount of probe DNAs on the substrate that were obtained in Example 3, a nucleic acid base (ddTTP-Cy5) with fluorescent dye being attached was modified on the 3'-terminal of the immobilised probe DNAs, by use of Terminal deoxynucleotidyl transferase, and the fluorescence intensity was measured by a fluorescence scanner, the intensity being obtained by excited the fluorochrome cy5.

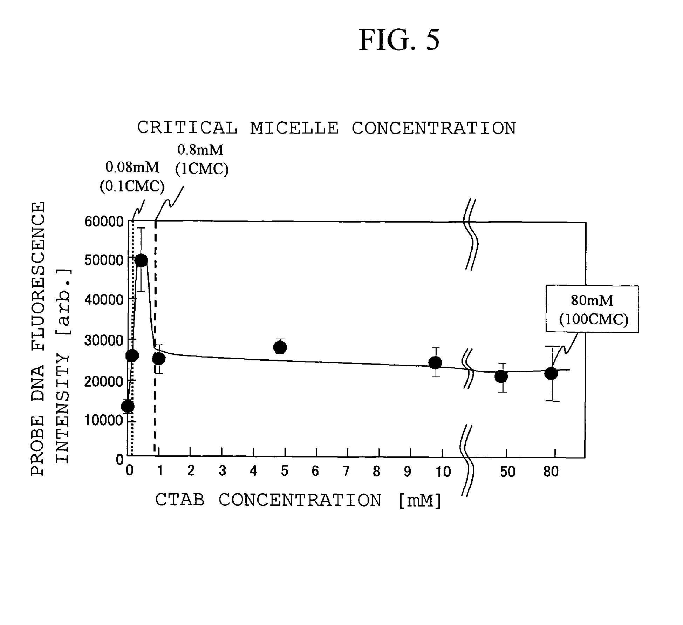

FIG. 5 shows a result of the above measurement. According to FIG. 5, it is found that the average amount of immobilised probe DNAs is increased in proportion to the added amount of CTAB. When the concentration becomes around 0.8 mM being the critical micelle concentration (CMC), the average amount of immobilised probe DNAs is almost maximised and becomes approximately four times compared to the case where the CTAB is not added. If the concentration of CTAB is increased further, the immobilised amount goes down.

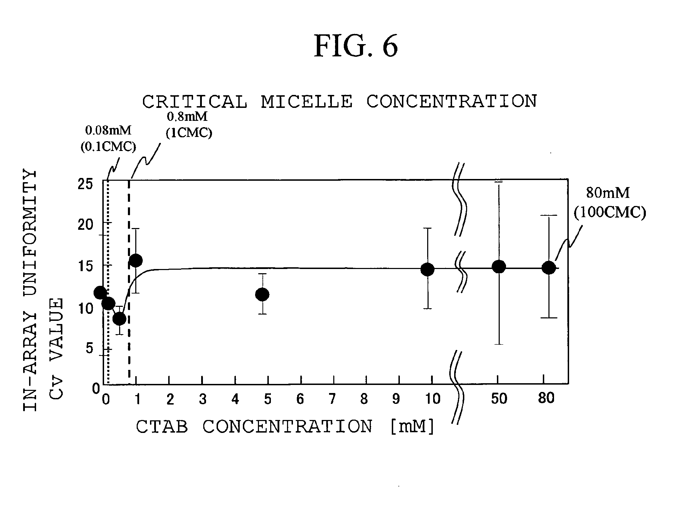

FIG. 6 shows the relationship between the uniformity in probe DNA immobilisation and CTAB addition concentration. The uniformity is expressed by a value of Cv (Coefficient of Variation) of the fluorescence intensity among a plurality of spots. It is found that when the CTAB addition concentration is around the CMC, the uniformity is enhanced. Therefore, it is further understood that adding CTAB with an appropriate concentration is effective for the uniformity of the probe DNAs.

Furthermore, the substrate obtained in Example 3 was used to measure the probe DNA amount which was non-specifically adsorbed when the CTAB was added. The Probe DNAs having amino groups on the terminal react with the reactive groups on the substrate surface to form a covalent bond, so as to be immobilised, but the probe DNA which does not have amino groups on the terminal does not form the covalent bond. Therefore, the attached amount of probe DNAs that do not have amino groups is equal to the amount non-specifically adsorbed. Here, the ratio of the non-specifically adsorbed amount is calculated. The ratio of the non-specifically adsorbed amount is obtained by use of the aforementioned fluorochrome (cy5), and it is the ratio of the immobilised amount of probe DNAs without amino groups on the terminal to the immobilised amount of probe DNAs having amino groups on the 5'-terminal. The probe DNAs used for the experiment have the same base sequence as a 50-mer probe DNA.

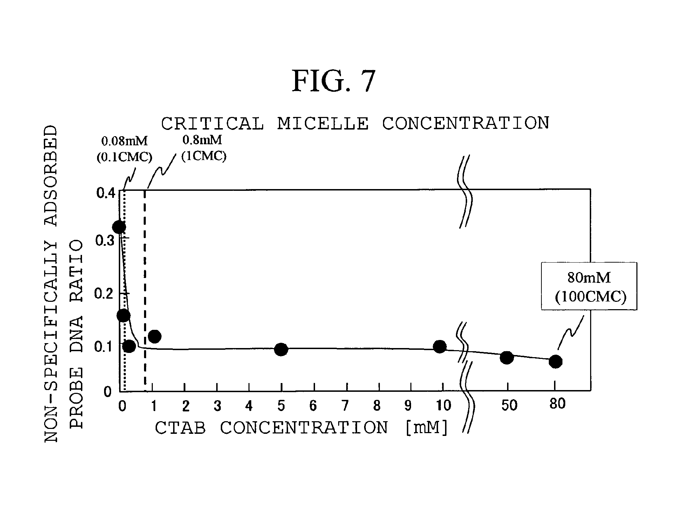

FIG. 7 shows the relationship between the CTAB concentration and the ratio of non-specifically adsorbed amount. It is found that by adding the CTAB, the ratio of non-specifically adsorbed probe DNAs can be reduced to 10% or less.

It is found according to the results so far described that in order to improve the reaction efficiency by approximately twice or more to immobilise the probe DNAs onto the substrate surface, it is desirable to add the CTAB with a concentration in the range of 0.1 CMC (0.08 mM) or more. As shown in FIG. 7, if the concentration is 0.1 CMC or more, it is also possible to reduce the ratio of probe DNAs which are non-specifically adsorbed.

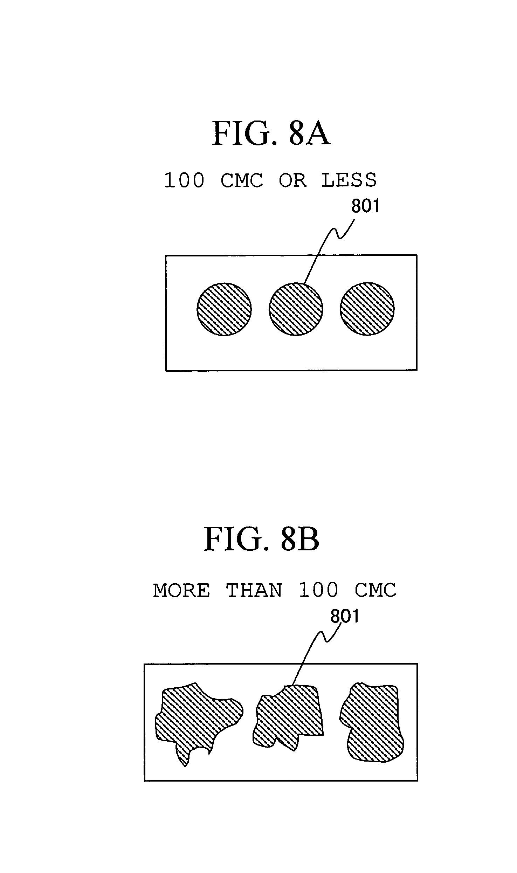

On the other hand, if the CTAB concentration is set high, such as more than 100 CMC (80 mM), the wettability between the substrate surface and the reaction solution is extremely increased, the spot shape may easily become distorted as shown in FIG. 8B, instead of symmetric circular. Therefore, it if found desirable that the CTAB concentration is set to 100 CMC or less.

The amount of solution spotted for immobilising the probe DNAs is around a few pL per spot, in the case of a small amount. Even under highly humid circumstances, moisture in the solution may evaporate and the CTAB concentrations may vary by spot. It is desirable in the above case that the immobilised probe DNA amount does not change drastically in response to the change of CTAB concentration.

According to FIG. 5, the range of CTAB concentration that does not cause a drastic change of the probe DNA immobilised amount is at least 1 CMC (0.8 mM), preferably 10 CMC (8mM) or more. Consequently, it is found that the addition of CTAB with the above concentration is desirable, in order to immobilise the probe DNAs within the range where the immobilised amount does not change so much depending on the CTAB concentration, to enhance the reaction efficiency, and further to reduce the probe DNAs adsorbed non-specifically.

### <EXAMPLE 5> Hybridisation

Quantity of Hybridisation as to the immobilised probe DNAs was evaluated. A target DNA was hybridised on the substrate on which the probe DNAs were immobilised by use of a solution in which CTAB with a concentration of 0.1 CMC = C = 100 CMC was added according to Examples 1 to 3, the target DNA being completely complementary with the probe DNA. 5x SSC (Standard Saline Citrate), 0.5% SDS solution (Sodium Dodecylsulphate) was used as the hybridisation solution, and after the hybridisation at 42°C, washing was performed with 2x SSC, 0.1% SDS solution and 1x SSC, 0.1% SDS solution. The terminal of the complete complementary target DNA is modified with a fluorescent molecule of Cy5.

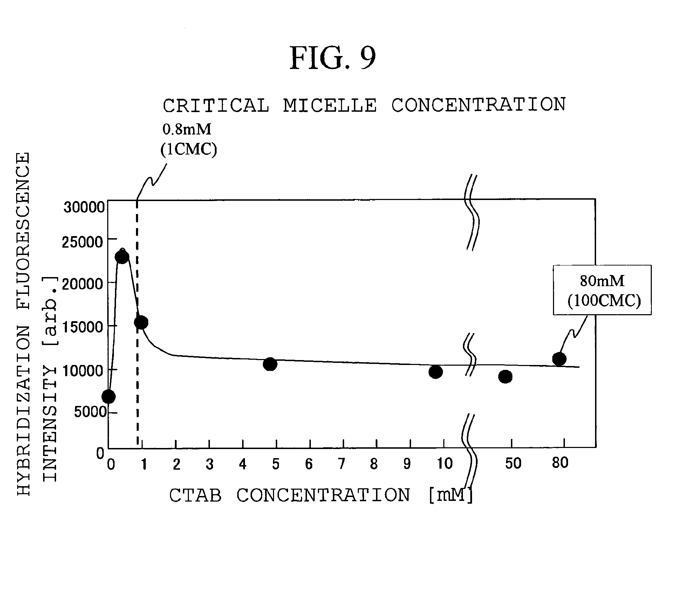

FIG. 9 shows the result of measured fluorescent intensity by use of the fluorescent scanner, after hybridisation. It is found that by immobilising the probe DNAs with a solution to which CTAB is added, the fluorescence intensity is increased after hybridisation.

### <EXAMPLE 6> Measurement of probe DNA density

Here, a result of detailed examination is shown as to the density of the probe DNAs which were immobilised by Example 3. The adherent amount of the probe DNAs was measured by use of an X-ray reflectance method, and the result was that the probe DNA density was equal to or more than 2 × 10¹² molecules/cm², when at least 0.1 CMC of CTAB was added.

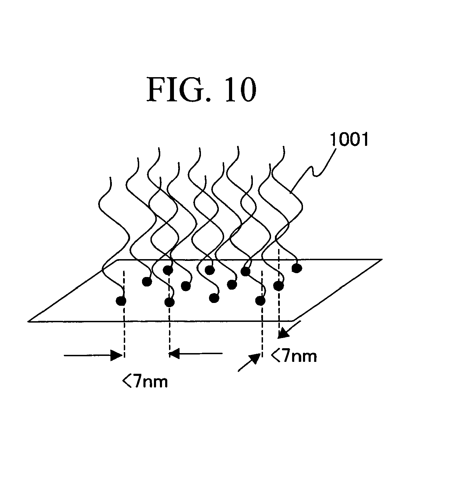

FIG. 10 is a schematic block diagram showing the substrate surface when the density of the probe DNA 901 is 2 × 10¹² molecules/cm². It is found that by adding CTAB at least 0.1 CMC, the density of probe DNAs is allowed to be equal to or more than 2 × 10¹² molecules/cm², that is, the distance between the two probe DNAs is allowed to be within 7 nm.

### <EXAMPLE 7> Influences of water content in reaction solution and reaction time, in the process of introducing amino groups

Influences of the water content in the reaction solution and the reaction time, when amino groups are introduced in Example 1, were examined. The Table shows the result of the examination as to the relationship between water content of the reaction solution, reaction time, the probe DNA uniformity, and its immobilised amount.

The probe DNA uniformity is expressed in Cv value of fluorescence intensity among spots within the array. When the water content in the reaction solution is less than 10%, the uniformity is deteriorated, and the immobilised amount is reduced. However, when the water content is in the range of large amount such as 10% or more, the immobilisation uniformity of the probe DNAs is improved, and the amount of DNA having been immobilised is large. As for the reaction time, it has been found that if the reaction time is one hour or more, the uniformity is deteriorated, and the immobilised amount is reduced.

Consequently, it is found desirable to perform amination with a reaction time of less than one hour, and with at least 10% water content of the reaction solution.

**TABLE**

| IMMOBILISED AMOUNT AND UNIFORMITY OF PROBE DNAs ACCORDING TO SILANE COUPLING REACTION CONDITIONS | | | | |
|---|---|---|---|---|
| Water content for amination | Reaction time for amination | Probe DNA immobilised amount fluorescence intensity [×10³ arb.] | Probe DNA immobilised uniformity [%] | Judgement |
| 0% | 5min | 5.2 | 14 | |
| | 30min | 5.0 | 14 | |
| | 1hr | 4.9 | 15 | |
| | 5hr | 3.9 | 17 | |
| 2% | 5min | 6.2 | 13 | |
| | 30min | 5.7 | 13 | |
| | 1hr | 5.5 | 14 | |
| | 5hr | 5.0 | 15 | |
| 10% | 5min | 12.5 | 12 | ○ |
| | 30min | 11.7 | 12 | ○ |
| | 1hr | 10.5 | 14 | |
| | 5hr | 9.5 | 15 | |
| 20% | 5min | 15.2 | 11 | ○ |
| | 30min | 14.0 | 12 | ○ |
| | 1hr | 13.1 | 13 | |
| | 5hr | 12.5 | 14 | |

### <Example 8>

In order to manufacture bead arrays for genetic analysis use, beads were employed instead of the substrate, and the beads on which probe DNAs were immobilised by Examples 1 to 3 were obtained. In addition, multiple types of beads were obtained by immobilising probe DNAs having one base sequence per bead. The probe DNAs were immobilised after the beads were soaked in the reaction solution. The material of the beads used here was borosilicate glass, and the bead diameter was around 100 µm.

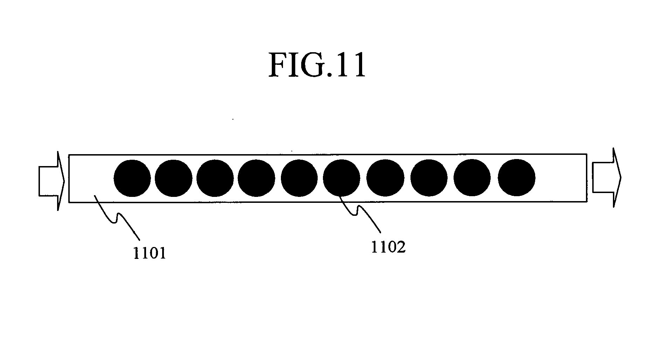

FIG. 11 shows a bead array for genetic analysis use, where 10 types of beads 1002 on which probe DNAs different from one another are immobilised, manufactured according to the above process, are accommodated in a micro channel 1001, thereby forming one array.

As for the beads described above, similar to the results shown in FIG. 5, FIG. 6, and FIG. 7, it was possible to improve the probe DNA immobilisation efficiency and to enhance the uniformity, by adding a positively charged surfactant as a phase transfer catalyst.

Furthermore, it was possible to reduce the non-specific adsorbed amount of probe DNAs. When the reaction time for the amination was set to within 1 hour, and the reaction solution water content was set to 10% or more, the probe DNA immobilisation efficiency and uniformity were improved, similar to the results shown in Table 1.

With the conditions above, probe DNAs were immobilised on the beads, and target DNAs having Cy5 as fluorescent molecules were hybridised, the target DNA being completely complementary with the probe DNA. At this stage, the fluorescence intensity and uniformity were examined. The fluorescence intensity was measured by means of a fluorescence scanner. As a comparative example, the reaction time was set to 5 hours and the water content was set to 2% as conditions for amination, and probe DNAs were immobilised on the beads under the condition that a surfactant was not added. Then, beads obtained according to the above process were also employed.

Consequently, in the case where the beads on which probe DNAs were immobilised under the conditions according to the present invention, higher fluorescence intensity after hybridisation occurs. This means that the target DNAs can be captured on the beads surface more efficiently. In addition, it is found that variation in fluorescence intensity between beads on which the same probe DNAs have been immobilised can be reduced.

While we have shown and described several embodiments in accordance with our invention, it should be understood that the embodiments disclosed are susceptible of changes and modifications without departing from the scope of the invention. Therefore, we do not intend to be bound by the details shown and described herein but intend to cover all such changes and modifications a fall within the ambit of the appended claims.

## Claims

1. A method for manufacturing a biosensor element in which probe biomolecules are immobilised on a substrate, wherein a surfactant is added to a reaction solution for immobilisation, when the probe biomolecules are immobilised on the substrate.

2. A biosensor element in which probe biomolecules are immobilised on a substrate, wherein a surfactant is added to a reaction solution for immobilisation, when the probe biomolecules are immobilised on the substrate.

3. The biosensor element of claim 2, wherein the density of the probe biomolecules is 2 × 10¹² molecules/cm² or more.

4. The biosensor element of claim 2, wherein the ratio of the probe biomolecules non-specifically adsorbed is 10% or less.

5. The method of claim 1 or the biosensor element of claim 2, wherein said probe biomolecules are nucleic acid and said surfactant is a positively charged surfactant.

6. The method of claim 1 or the biosensor element of claim 2, wherein when the probe biomolecules are protein and the effective charge of the protein molecules is negative, a positively charged surfactant is used as said surfactant, whereas a negatively charged surfactant is used when the effective charge is positive.

7. The method of claim 1 or the biosensor element of claim 2, wherein the concentration C of said surfactant is 0.1 CMC = C = 100 CMC (CMC: critical micelle concentration).

8. The method of claim 1 or the biosensor element of claim 2, wherein the concentration C of said surfactant is at least 1 CMC (CMC: critical micelle concentration).

9. The method of claim 1 or the biosensor element of claim 2, wherein, when said probe biomolecules are immobilised and said biomolecules are immobilised through the intermediary of silane coupling agent molecules, the water content of the reaction solution when the silane coupling agents are allowed to react with the substrate surface is at least 10%, and the reaction time is equal to or less than one hour.
